# EUROPÄISCHE PATENTANMELDUNG

(11) **EP 1 308 128 A2**
(43) Veröffentlichungstag der Anmeldung: **07.05.2003**
(21) Anmeldenummer: 02023999.2
(22) Anmeldetag: 26.10.2002
(51) Int. Cl.: A61B 5/103

(54) **Vorrichtung und Verfahren zur Messung der Refraktion eines Auges**

(30) Priorität: 01.11.2001 DE 10153397
(71) Anmelder: plusoptiX AG, 90411 Nürnberg (DE)
(72) Erfinder: Bräuning, Johannes, Dr., 73760 Ostfildern (DE)
(74) Vertreter: Patentanwälte , Ruff, Wilhelm, Beier, Dauster & Partner

(57) **Zusammenfassung**

Die Erfindung bezieht sich auf eine Vorrichtung und ein Verfahren zur Refraktionsbestimmung aus einer relativen Distanz unter Verwendung einer Kamera (51), einer Beleuchtungseinheit (52), einer Blendeneinheit und einer Steuereinheit (52,54,55).

Erfindungsgemäß ist eine dynamische Helligkeits- und/oder Beleuchtungszeiteinstellung für die Beleuchtungseinheit und/oder eine Erfassung der Patientendistanz und/oder eine selbsttätige Messbereichskontrolle vorgesehen.

Verwendung bei der Refraktionsbestimmung.

## Beschreibung

Die Erfindung bezieht sich auf eine Vorrichtung und ein Verfahren zur Refraktionsbestimmung. Die Erfindung zielt insbesondere auf die Möglichkeit einer genaueren Messung und verbesserten Automatisierung des Messvorganges ab. Zudem wird das Problem der Darbietung eines Anreizes zur Blickrichtungskoordination gelöst.

Die exzentrische Photorefraktion ist ein Verfahren, mit dessen Hilfe man die Refraktion eines Auges aus einer Distanz messen kann. Daher ist diese Meßmethode insbesondere geeignet für nicht kooperative Patienten, wie z.B. Kleinkinder. Ein herkömmlicher Aufbau eines Photofraktionssystems bzw. Videofraktionssystems ist z.B. aus der Patentschrift DE 197 19 694 C2 bekannt. Das Meßsystem besteht aus einem bildaufnehmenden System (wie z.B. Videokamera) sowie einem speziellen Beleuchtungssystem, das im wesentlichen durch eine spezielle Anordnung von Leuchtdioden (Infrarot) um ein Objekt herum gekennzeichnet ist. In Korrespondenz zu den Leuchtdiodenfeldern ist am Objektiv eine Abdeckblende angebracht, die einen Teil des ausgesendeten, vom Auge rückreflektierten Lichtes abblockt. Dadurch entsteht in der Pupille des Auges ein Helligkeitsprofil, das nach Analyse (z.B. Regression) mit einem Refraktionswert korreliert werden kann. Durch die Kombination mehrerer Blenden und LED-Felder lässt sich die Refraktion des Auges in verschiedenen Achsen bestimmen und somit die volle Refraktion bestehend aus Sphäre, Zylinder und Achslage messen. In der genannten DE 197 19 694 C2 wird eine Vorrichtung vorgestellt, die bestehend aus einem Kamerasystem, einer Blende, einem Beleuchtungssystem und einem Monitorsystem ein derartiges Refraktionsgerät darstellt. Die Messung der Refraktion ist mit diesem Gerät in Echtzeit (Videosystem) möglich.

Ein Problem der bisher bekannten Verfahren ist die unterschiedliche Reflektivität der Lichtquelle an der Netzhaut und dadurch ein unterschiedlicher Helligkeitsverlauf in der Pupille. Weiterhin ist das in das Auge einfallende Licht abhängig von der Pupillengröße. Die genannten Verfahren sehen hierfür keine Anpassungsmöglichkeiten vor. Ebenso sind die genannten Verfahren empfindlich gegenüber der Nichteinhaltung der vorgegebenen Distanz. Weiterhin ist das Problem der Darbietung eines Targets zur Definition der Blickrichtung für ein Distanzmessverfahren nicht gelöst. Hierbei ist insbesondere nicht gelöst, inwiefern man ein Target für verschiedene "virtuelle Distanzen" darbieten kann. In diesem Zusammenhang sind auch keine Verfahren bekannt, um ein Auge aus der Distanz zu "vernebeln".

Der Erfindung liegt als technisches Problem die Bereitstellung einer Vorrichtung und eines Verfahrens zur Refraktionsbestimmung zugrunde, mit denen sich die oben genannten Problempunkte herkömmlicher Systeme wenigstens teilweise beheben lassen und die insbesondere eine Helligkeitssteuerung zur Anpassung an unterschiedliche Beleuchtungsanforderungen, eine Patientendistanzkontrolle und/oder eine selbsttätige Messbereichskontrolle, ob sich die Sehcharakteristik des Patienten bzw. Probanden innerhalb des Messbereichs des Systems befindet, ermöglichen.

Die Erfindung löst dieses Problem durch die Bereitstellung einer Vorrichtung zur Refraktionsbestimmung mit den Merkmalen des Anspruchs 1, 2 oder 3 und eines entsprechenden Verfahrens mit den Merkmalen des Anspruchs 8, 9 oder 10.

Die erfindungsgemäße Vorrichtung und das erfindungsgemäße Verfahren leisten eine automatisierte Anpassung des Messvorganges an das Auge. Das Problem der unterschiedlichen Beleuchtungsanforderung bei unterschiedlich weiter Pupille sowie bei unterschiedlicher Reflektivität der Netzhaut, das zu einer uneinheitlichen Reflektion des Pupillenprofils führt, wird durch eine geeignete Helligkeitssteuerung gelöst. Diese beruht auf der Steuerung der Beleuchtungsintensität in Abhängigkeit von der Reflektion der Netzhaut unter den unterschiedlichen genannten Bedingungen. Hierfür ist sowohl ein Regelkreis als auch eine Steuerung vorgesehen. Ebenso wird das Problem der Darstellung eines Targets (Blickzielobjekt) gelöst. Das Target kann "virtuell" auf verschiedene "Distanzen" des Auges eingestellt werden. Dies macht es möglich, den Akkommodationszustand entsprechend den Targeteinstellungen zu beeinflussen und somit Akkommodationsmessungen durchführen zu können.

Mit einer Regelung der Helligkeit der z.B. aus einer LED-Anordnung bestehenden Beleuchtungseinheit ist eine Adaption auf unterschiedliche Pupillengrößen möglich, ohne in einen Bereich der Übersteuerung in der Reflektion eines Auges zu gelangen. Die Helligkeitsregelung kann selbsttätig in Abhängigkeit von entsprechenden Messdaten erfolgen.

Gemäß einem weiteren Erfindungsaspekt kann eine Distanzkontrolle implementiert sein, mit der die Distanz des Patienten zum Messgerät erfasst wird. Zum einen erleichtert dies die genaue Positionierung eines Probanden, zum anderen können Messdaten der Refraktionsbestimmung bei verschiedenen, gemessenen Patientendistanzen aufgenommen und geeignet ausgewertet werden.

In einem weiteren Aspekt der Erfindung ist eine Messbereichskontrolle vorgesehen, durch die selbsttätig erkannt wird, ob sich ein Proband mit seiner Sehcharakteristik außerhalb des Gerätemessbereichs von z.B. +5 Dioptrien bis -7 Dioptrien befindet. Zur Durchführung einer solchen Messbereichskontrolle kann ein System dienen, das auf dem Vergleich von Messungen beruht, die bei unterschiedlicher Beleuchtung durchgeführt worden sind. Ein positives Ergebnis der Messbereichskontrolle ist dann dadurch definiert, dass es möglich ist, verschiedene Beleuchtungsmuster z.B. durch eine LED-Anordnung ansteuern zu können.

Vorteilhafte Weiterbildungen der Erfindung sind in den Unteransprüchen angegeben.

Vorteilhafte Ausführungsformen der Erfindung sind in den Zeichnungen dargestellt und werden nachfolgend beschrieben. Hierbei zeigen:
- Fig. 1: eine schematische Draufsicht auf eine Vorrichtung zur Refraktionsbestimmung,
- Fig. 2a: eine schematische Seitenansicht eines Messkopfteils mit Kamera der Vorrichtung von Fig. 1,
- Fig. 2b: eine Draufsicht auf einen Retinoskop-Systemteil mit Beleuchtungsfeld- und Blendenanordnung für die Vorrichtung gemäß Fig. 1 und 2a,
- Fig. 3a: eine schematische Seitenansicht eines Targetsystemteils für die Vorrichtung von Fig. 1 in einer Linsenoptikausführung,
- Fig. 3b: eine Ansicht entsprechend Fig. 3a, jedoch für ein Targetsystem in einer Spiegeloptikausführung,
- Fig. 4a: eine schematische Seitenansicht eines Targetsystems in einer Head-Up-Display-Technik,
- Fig. 4b: eine schematische Seitenansicht eines weiteren Targetsystems in einer Hohlspiegelausführung,
- Fig. 5: eine schematische Darstellung eines Regelkreises zur dynamischen Beleuchtungseinstellung für die Vorrichtung von Fig. 1 und
- Fig. 6: eine schematische Draufsicht auf eine in einem kompakten Gehäuse untergebrachte Vorrichtung nach Art von Fig. 1.

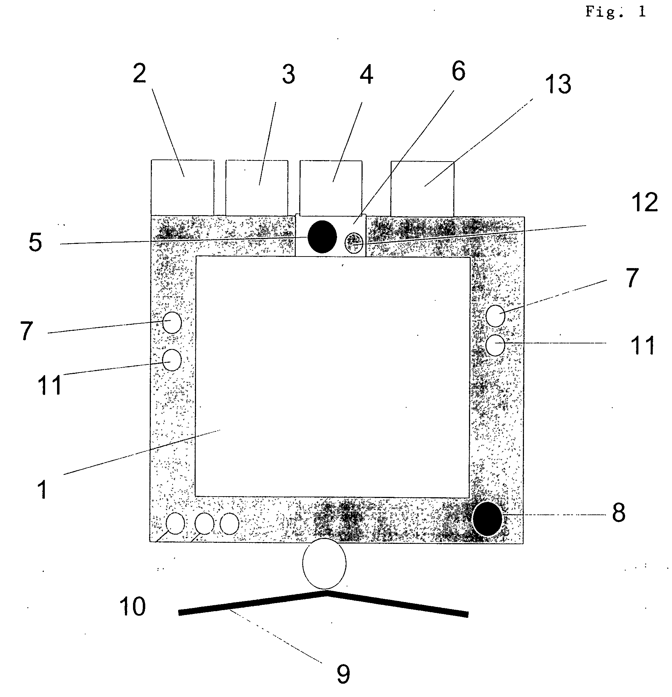

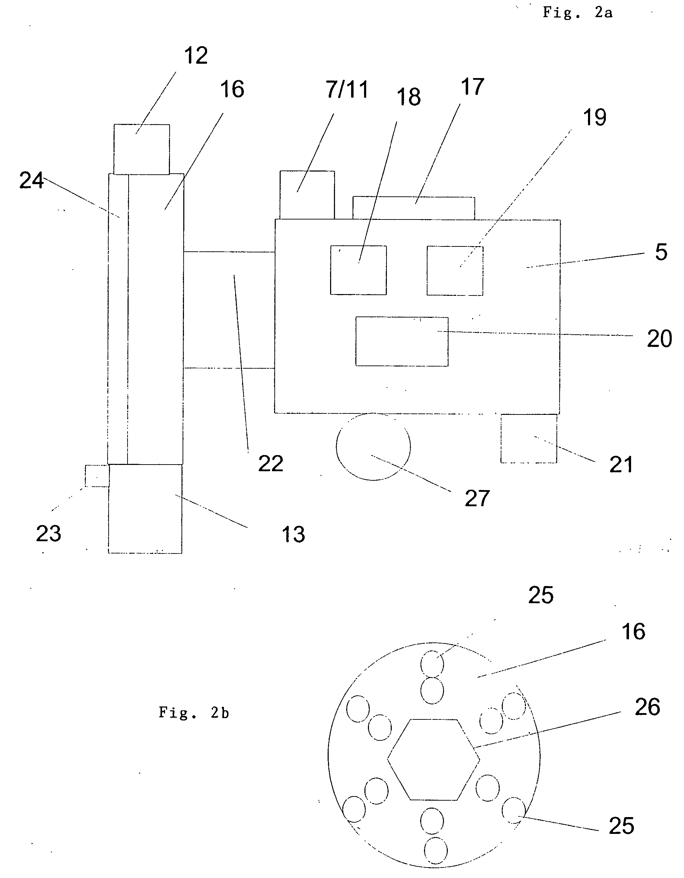

Fig. 1 stellt schematisch ein Refraktionssystem der vorbeschriebenen Art dar. Das System besteht aus einer Rechnereinheit, z.B. einem PC, sowie einem Anzeigeschirm 1. Diese Einheit ist auf einem Sockel 9 montiert, der z.B. mit einem Kugelgelenk zur Verkippung und Drehung der Einheit verbunden ist. An der Einheit befindet sich die zur Messung notwendige Kamera 5 mit Objektiv und Beleuchtungseinheit und Blende, wie aus Fig. 2a detaillierter ersichtlich. Die Kamera mit der dazugehörigen Elektronik und Beleuchtung kann sowohl in digitaler Ausführung als auch bekannter analoger Ausführung angebracht sein. Eine digitale Ausführung wäre beispielsweise über einen Schnittstellenstandard wie IEEE 1394 oder USB realisierbar. Eine analoge Ausführung beinhaltet einen Analog-Digital-Wandler, der die Videodaten für den PC zugänglich macht (Framegrabber). Ebenso findet sich in einer analogen Anwendung ein Digital-Analog-Wandler, der die Steuersignale des PCs in analoge Signale umsetzt, um beispielsweise die Helligkeit von LEDs zu steuern.

Am Gehäuse bzw. an der Kamera 5 befindet sich eine Messeinheit zur Bestimmung des Abstandes zwischen Gerät und Patient. Diese Messeinheit kann sich frontal am Gerät oder rückseitig am Gerät befinden. Dies ist abhängig von der Ausrichtung der Kamera (die Kamera kann nach vorne oder hinten ausgerichtet sein). Die Abstandsmessung kann optisch oder akustisch erfolgen. Eine akustische Messung ist vorzugsweise mit einem Ultraschallsender und einem Ultraschallempfänger 11 realisierbar. Dabei kann der Empfänger und Sender sowohl am PC-Gehäuse als auch an der Kamera selbst montiert sein. Ein optischer Distanzmesser 7 kann z.B. in der Form eines sichtbaren oder unsichtbaren (Infrarot-)Sensors oder Anzeigers realisiert sein. Im Falle des Sensors würde eine Sende- und Empfangseinheit automatisch (mittels Reflektion) die Distanz gegenüber dem Patienten messen. Im Falle einer Projektionseinheit kann z.B. ein Muster auf den Probanden projiziert werden (z.B. Stirn) und dieses Muster dann bei einer sichtbaren Lichtquelle direkt beurteilt oder im Falle einer unsichtbaren Lichtquelle durch die bildaufnehmende Kamera und die nachgeschaltete Software weiter verarbeitet werden. Mit Hilfe der zwei Projektionssysteme 7 kann durch Triangulation der Abstand eines Patienten vor dem Gerät sowohl berechnet als auch eingestellt werden.

Die durch die Distanzmessung gewonnenen Messdaten können auf zweierlei Art und Weise verarbeitet werden. Zum einen können die Daten in eine Anweisung umgesetzt werden, den Patienten ordnungsgemäß vor dem Gerät zu positionieren, zum anderen können die Distanzinformationen aktiv in das Messprogramm aufgenommen werden, um z.B. die Refraktionsmessung an die gewonnenen Abstandsdaten anzupassen. Dieses System ist insofern optimierbar, als dass die Blende der Kamera mit in das Regelkreissystem eingebracht werden kann. Wird an der Kamera ein großer Blendenwert eingestellt (kleiner physikalischer Durchmesser), so kann mit dem Kamerasystem eine große Schärfentiefe erreicht werden. Das Bild ist somit über einen größeren Distanzbereich scharf und kann damit berechnet und weiter verarbeitet werden. Durch die von den Distanzmeßsystemen gewonnenen Daten über die Entfernung des Patienten vom Gerät können in Korrelation zum Bild Messdaten (bei scharfem Bild) über einen größeren Distanzbereich gewonnen werden und entsprechend der gemessenen Distanz berechnet werden. Somit ist aus der Kombination Kamera, relativ großer Blendenwert und Distanzmeßsystem eine Art "Autofokus" zur Refraktionsmessung mittels exzentrischer Photorefraktion realisiert. Umgekehrt lässt sich das Distanzmeßsystem bei keinem Blendenwert (großer physikalischer Blendendurchmesser) als Hilfsinstrument zur geeigneten Positionierung des Patienten bei korrekter Distanz vor dem Meßsystem nutzen.

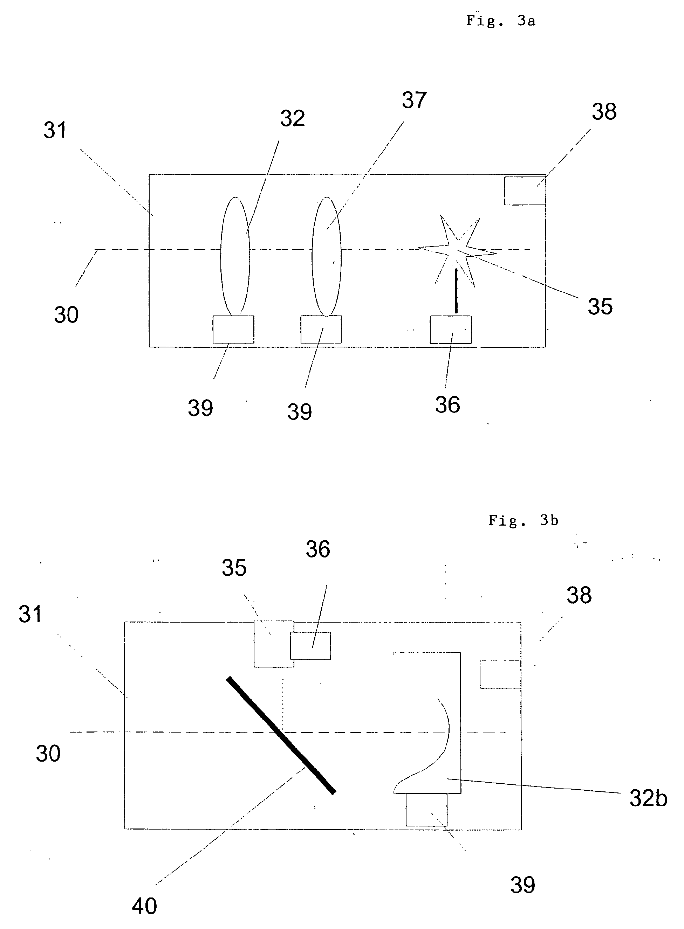

Weiterhin ist am Meßsystem die Kamera in drei Achsen bewegbar bzw. verkippbar und drehbar. Ebenso kann die Kamera abgenommen werden und ist dann mittels eines Kabels oder einer Sende-/Empfangseinheit 2 mit dem System verbunden. Weiterhin kann das System mit einem Blitz 4 ausgestattet werden, um die Größe der Pupillen des zu vermessenden Probanden zu beeinflussen. Weiterhin kann das System mit einem Raumsensor 3 zur Helligkeitsmessung des Raumes bzw. zur Aufdeckung von Infrarotstörstrahlern ausgestattet werden. Das System kann weiterhin mit einem sichtbaren oder unsichtbaren Zielstrahl 12 und der dazugehörigen Beleuchtung und Optik zur Einstellung des Patienten vor dem System ausgestattet sein. Weiterhin beinhaltet das System ein Target 13, das in Verbindung mit Fig. 3 noch näher beschrieben wird. Ebenso ist es denkbar, vor der Kamera einen Spiegel anzubringen, der dem Patient sein eigenes Spiegelbild darstellt. Der Spiegel muss für sichtbares Licht reflektiv und für Infrarotlicht penetrierbar sein. Befindet sich der Spiegel direkt vor der Kamera, so wird dem Probanden bei Betrachtung die doppelte Distanz am Messgerät mit entsprechender Beeinflussung der Akkommodation vorgetäuscht. Weiterhin sind am Gerät Steuerknöpfe 10 zur Bedienung angebracht. Dies kann auch in Form eines sogenannten Touchscreen-Schirmes realisiert sein.

Fig. 2a stellt in der Übersicht schematisch verschiedene Funktionen und Module der Kamera dar. Die Kamera kann sowohl in das Gesamtsystem integriert sein, als auch einen "mobilen Messkopf" darstellen. Der Messkopf kann im Falle der Ablösung vom Gesamtsystem mittels einer Funkoder Kabelverbindung mit dem PC-System verbunden sein. Es ist auch denkbar, die Funktionalität des PC-Systems in eine entsprechende vereinfachte Prozessortechnik zu integrieren und in die Kamera zu implementieren. Der Messkopf ist grundsätzlich aus dem sogenannten Retinoskop 16, dem Objektiv 22 und der Kamera 5 selbst aufgebaut. Dabei können sowohl das Objektiv der Kamera als auch die LEDs mit Filtern (z.B. Interferenzfilter, Polfilter, Lambda/4-Plättchen) ausgestattet sein.

Das Retinoskop beinhaltet Beleuchtungsfelder 25, die in der Regel mit Infrarot-LEDs ausgestattet sind. Im Beispiel von Fig. 2b besitzt das Retinoskop 16 sechs Felder 25, denen je eine Kante bzw. Blende 26 zugeordnet ist. Die Einheit aus Beleuchtungsfeld 25 und Blende 26 wird als Retinoskop 16 definiert. Das Gesamtsystem wird als Messkopf definiert.

Der Messkopf kann mit verschiedenen Features ausgestattet sein, die bereits in Fig. 1 dargestellt wurden. Über ein Drehgelenk 27 kann der Messkopf mit der PC-Einheit aus Fig. 1 verbunden sein. Das Objektiv 22 beinhaltet eine manuelle oder automatisch (softwaregesteuert) justierbare Blendeneinstellung. Mit dem Messkopf verbunden ist das Targetsystem 13. Alternativ dazu kann das Retinoskop 16 von einem Spiegel überdeckt sein, der sichtbares Licht reflektiert und unsichtbares Licht (Infrarot) durchlässt. Zum verbesserten Auffinden des Patienten mit der Kamera kann ein optischer Sucher 21, ein Zielstrahl mit entsprechender Optik 12 und/oder ein Display 17, das das aktuelle Kamerabild darstellt, dienen. Ebenso ist am Kamerakopf ein Abstandsmesser 7 bzw. 11, wie beschrieben, angebracht. Ein Sender 18 kann dabei bei einer kabellosen Version sowohl Bild- als auch Steuersignale zum PC schicken. Der Messkopf ist ebenso mit einem Taster 19 zur Bedienung des Messkopfs ausgestattet. Ein Griff 20 dient zur besseren Handhabung.

Die Fig. 3a und 3b stellen Beispiele für Einheiten zur Realisierung eines Targets bzw. zur Realisierung einer Vernebelungsoptik dar. Die Einheiten sind in einem Gehäuse untergebracht. Fig. 3a stellt eine Linsenoptik dar, Fig. 3b stellt eine Spiegeloptik dar.

In Fig. 3a sind verschiedene Objekte zur Realisierung entlang einer optischen Achse 30 angebracht. Durch eine Frontscheibe 31 wird der Einblick auf ein refraktives Element (z.B. Linse) 32 ermöglicht. Im weiteren Verlauf der optischen Achse kann sich ein zweites refraktives Element 37 befinden. Durch die refraktiven Elemente wird der Blick auf ein Fixationsobjekt 35 ermöglicht. Alle Einheiten entlang der optischen Achse können mechanisch bewegt werden. Die refraktiven Elemente können z.B. durch eine Spindelmechanik 39 jeweils einzeln bewegt werden. Ebenso kann das Objekt 35 mittels einer Mechanik 36 bewegt werden. Die Bewegung von Optik oder Mechanik ist nötig, um das Objekt virtuell in verschiedenen Entfernungen darzubieten bzw. die Akkommodation des Auges entsprechend zu beeinflussen. Mit Hilfe der beweglichen Komponenten ist auch eine "Vernebelung" möglich. Um chromatische Abberationen weitgehend zu eliminieren, kann innerhalb des Gehäuses eine monochromatische Beleuchtungsquelle 38 verwendet werden. Alternativ dazu kann auch das Objekt 35 selbst monochromatisch beleuchtet werden. Alternativ zur Bewegung der refraktiven Elemente bzw. des Objektes ist es möglich, ein Objekt zu implementieren, das in seiner räumlichen Ausdehnung aus verschiedenen Beleuchtungseinheiten besteht. Dies kann z.B. in einer LED-Reihe oder in LED-Ringen realisiert werden, die sich entlang der optischen Achse ausdehnen. Eine Pseudo-Verschiebung wäre somit durch das Anschalten bzw. Beleuchten der Einzelobjekte entlang der optischen Achse möglich.

Fig. 3b stellt eine alternative Lösungsmöglichkeit dar. Das refraktive Element besteht hier nicht in Form von Linsen, sondern in Form eines Spiegels 32b (Konkavspiegel). Auch hier ist der Spiegel 32b über eine Mechanik 39 entlang der optischen Achse 30 verschiebbar. Ein Strahlteiler 40 ist derart ausgebildet, dass beim Einblick ein Fixationsobjekt 35 über den Spiegel 32b eingesehen werden kann. Auch dieses Objekt 35 ist mit einer Mechanik 36 beweglich. Ebenso wie in Fig. 3a ist anstatt eines mechanisch bewegbaren Objekts eine LED-Reihe etc. denkbar. Ebenso wie in Fig. 3a gelten auch hier monochromatische Lichtverhältnisse 38. Alternativ zur Bewegung des Spiegels ist auch ein adaptiver Spiegel denkbar, der in seiner Brechkraft justierbar ist. Derartige Spiegel werden derzeit in sogenannte adaptive Optiken implementiert.

Alternativ kann in den Strahlengang auch ein Target aus IRtransparentem Material eingebracht werden. Wird das Target mit einem Handgriff versehen, so kann es frei gehalten werden. Eine automatische Positionsbestimmung kann dadurch erfolgen, dass das Target ebenso mit IR-reflektivem oder IR-absorbierendem Material versehen wird. Bei Aufbringung von mindestens zwei derartigen Objekten ist mittels softwareunterstützter Objekterkennung und Triangulation die Position des Targets im Raum automatisch bestimmbar.

Allgemein kann in einem Autorefraktor die Akkommodationsbreite bestimmt werden, indem das Auge mittels einer Optik (auch virtuell) mit einem Objekt aus nächster Nähe bis ins Unendliche und darüber hinaus ("Nebel") konfrontiert wird. Dabei wird der Brechzustand in kurzen Intervallen gemessen. Dies führt zu einer Refraktionszustandsverteilung, die in der Nähe und der Ferne gemäß den individuellen Gegebenheiten in eine Plateauphase führt. Die Differenz der Plateauphasen (Dioptrien) entspricht der Akkommodationsbreite, die mit diesem Verfahren automatisch gemessen werden kann.

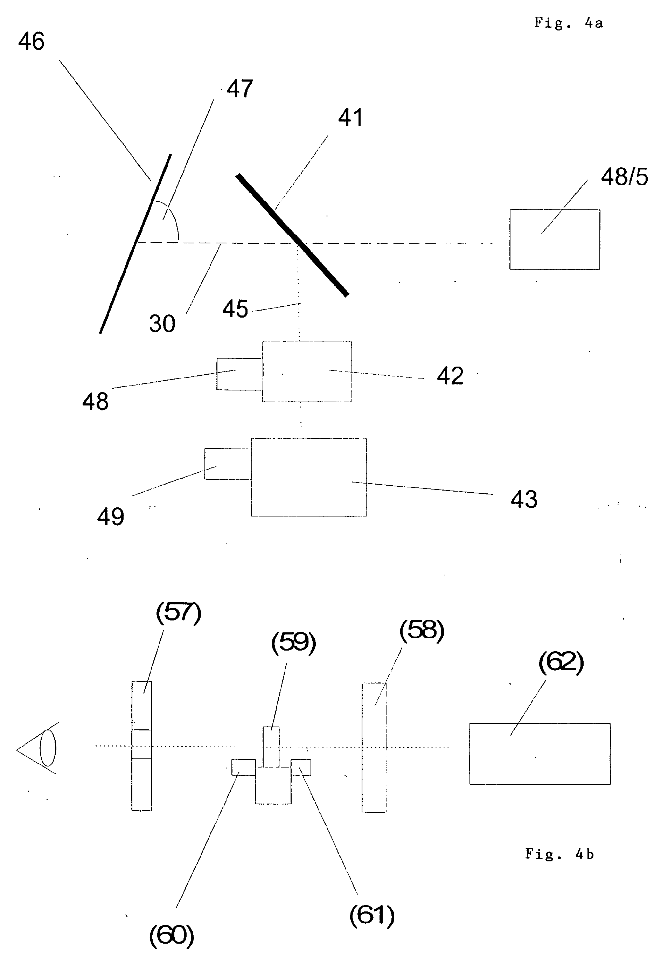

Während in den Fig. 3a und 3b eine Einblickstarget-Lösung vorgestellt wurde, wird in Fig. 4a eine Target-Lösung vorgestellt, die mittels einer Head-up-Display-Technik ein Blickzielobjekt anbietet. Bei der Head-up-Display-Technik kann zwar ein primitives Zielobjekt als Target benutzt werden, sie ist aber eher zur Nutzung des Displays als Targeteinheit gedacht. Vorteil dieses Verfahrens ist es, dass die Echtzeitmessung, die auf dem Display dargestellt wird, direkt vom Patienten eingesehen werden kann und gleichzeitig als Target zur Erzeugung in verschiedenen virtuellen Distanzen benutzt werden kann. Fig. 4a zeigt den prinzipiellen Aufbau eines solchen Systems. Ein entsprechender Spiegel 41, der transparent für Infrarotlicht ist, wird in die optische Achse 45 eingebracht. Eine in der optischen Achse befindliche Frontscheibe 46 ist um einen Winkel 47 in Relation zur optischen Achse der Kamera mit Retinoskop 48/5 verkippt, um Reflektionen aus der vom Messkopf kommenden Beleuchtung zu vermeiden. Entlang der Spiegelachse 45 befindet sich ein refraktives Element 42 mit einer mechanischen Verstellung 48 entlang der Spiegelachse 45. In die Spiegelachse 45 ist ein Targetsystem 43 eingebracht, das ein Objekttargetsystem oder ein Display 1 darstellen kann. Auch dieses System ist entlang der optischen Achse 45 mit Hilfe einer Mechanik 49 bewegbar. In dieser Anordnung ist der Spiegel 41 für sichtbares Licht nicht transparent, lediglich ist er transparent für IR-Licht.

In einer Modifikation von Fig. 4a ist es denkbar, einen Spiegel 41 zu verwenden, der für sichtbares Licht transparent ist. Dies könnte z.B. eine Art Autoglasscheibe für Head-up-Displays sein. Bringt man die Elemente entlang der Achse 45 in die Achse 44 ein, so blickt der Proband direkt auf die Projektionseinheit bzw. das refraktive Element 42 und/oder das Targetsystem bzw. Display 43. Hierbei ist es möglich, das Messkopfsystem 48 entlang der optischen Achse 44 in Exzentrizität zu belassen, oder alternativ in die optische Achse 45 einzubringen. In diesem Falle würde man einen Kaltlichtspiegel verwenden. Bringt man alle Elemente 42, 43, 48 entlang der optischen Achse 44 ein, so könnte entlang der Achse 45 ein weiteres Target zusätzlich z.B. entsprechend der Fig. 3a oder 3b eingebracht werden.

Eine weitere Target-Lösung stellt Fig. 4b dar. Eine Öffnung in einem Hohlspiegel 57 dient als Einblicköffnung für den Patienten. Ein bewegliches Objekt 59 wird durch ein optisches System 58, das z.B. aus verschiebbaren Hohlspiegeln besteht, auf die Netzhaut abgebildet. Das Objekt kann wahlweise von jeder Seite durch die Beleuchtungseinheit 60, 61 beleuchtet werden, so dass mit einer Objektposition zwei Targetpositionen visualisiert werden. Durch den IR-transparenten Hohlspiegel erfolgt die Refraktionsmessung mit dem Refraktor 62.

Eine weitere Target-Lösung stellen Hologramme dar, auf denen Objekte aufgenommen werden, die für den Betrachter in verschiedenen Abständen virtuelle Objekte darstellen. Solche Objekte können neben alltäglichen Gegenständen auch optische Systeme, wie ein Fernrohr, enthalten. Durch den Blick durch ein holografisch aufgenommenes optisches System können dann z.B. Objekte in beliebiger Entfernung (z.B. Nahbereich und unendlich) sowie Vernebelung realisiert werden.

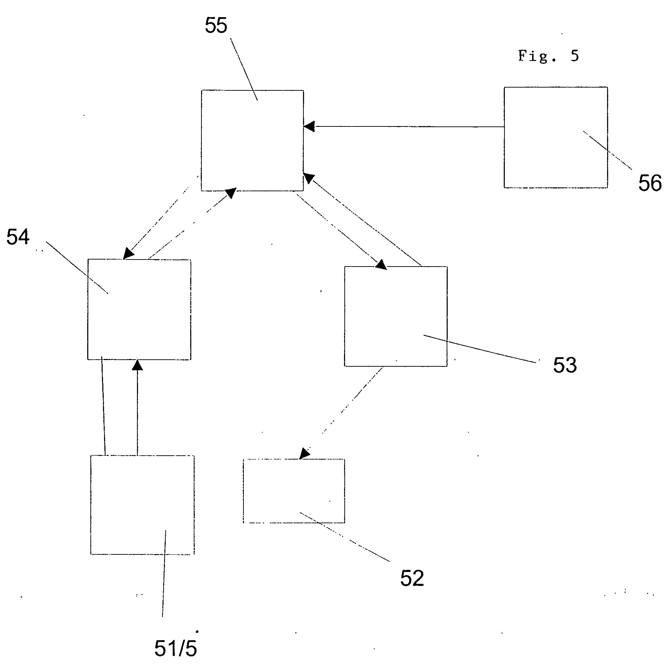

Fig. 5 stellt einen Regelkreis für die dynamische Beleuchtungseinstellung dar. Durch die dynamische Beleuchtungseinstellung mit Einstellbarkeit der Intensität und Einstellbarkeit der Beleuchtungsdauer der Retinoskop-Beleuchtung kann auf eine manuelle Einstellung der Blendenöffnung an der Kamera weitgehend verzichtet werden. Der im folgenden beschriebene Regelkreis ist abhängig von der Fokussierung des Gesamtsystems und ist somit nur kontinuierlich aktiv, wenn die Fokussierung des Gesamtsystems gewährleistet ist. Diese Gewährleistung der Fokussierung kann durch das Abstandsmeßsystem 56/11/7 gemessen werden. Der Regelkreis beinhaltet eine Standardeinstellung für die LED-Intensität, die den Startwert oder Rückstellwert definiert. Die Kamera 51/5 aktiviert ein Bild, das in den Bildspeicher des PCs 54 weitergegeben wird. Eine Auswertung des Bildes bezüglich der Helligkeitsqualität erfolgt durch eine CPU 55. Entsprechend der ermittelten Helligkeit wird ein Steuersignal zur LED-Helligkeitssteuerung 53 erzeugt. Das Steuersignal kann sowohl eine hoch- oder niederfrequente Pulslänge als auch deren Periodizität oder Amplitude (Stromintensität) bestimmen. Die Steuerung setzt dieses Signal in ein Signal für die Beleuchtungsdauer und/oder die Beleuchtungsintensität der LED 52 um. Das erneut durch die Kamera 51/5 aufgenommene Bild wird erneut im Bildspeicher 54 ausgewertet usw.

Ebenso ist es möglich, die Beleuchtungszeit der Kamera bei diesem Regelkreis zu steuern. Um diesen Regelkreis nicht ins "Schwingen" geraten zu lassen, wird zusätzlich ein Signal von der Abstandsmesseinrichtung integriert. Lässt sich aus diesem Signal ableiten, dass das Objekt im Fokus der Kamera liegt, so wird die LED-Helligkeit weiterhin bis an ein vorgegebenes Optimum angepasst. Ist aus dem Abstandsmesssignal ableitbar, dass sich das Objekt außerhalb des Fokus der Kamera befindet, wird der Regelkreis unterbrochen und auf einen Standardwert zurückgesetzt. Steht ein System zur Verfügung, das über kein Abstandsmesssignal verfügt, so wird der Regelkreis in bestimmten Intervallen aktiviert und deaktiviert, um einer Schwingung vorzubeugen. Die LED-Felder können in ihrer Intensität und Ansteuerungszeit als ganzes Feld, aber ebenso auch einzeln nach verschiedenen Mustern angesteuert werden.

Dies kann in Abhängigkeit zur gemessenen Pupillengröße oder zum Reflektionsprofil (z.B. mittlere Helligkeit) erfolgen. Dabei können die LEDs bzw. die Beleuchtung mit gleicher oder unterschiedlicher Leistung betrieben werden. Im Falle einer linearen radialen Anordnung würde beispielsweise die Intensität der Helligkeit mit zunehmender Exzentrizität zunehmen. Ebenso ist ein weiteres LED-Feld außerhalb der LED-Felder des Retinoskopes am Gehäuse oder an der Kamera anbringbar. Dieses LED-Feld wird zur Vergleichsmessung eines Referenz-LED-Feldes innerhalb des Retinoskopes dahingehend genutzt, dass die Linearität des Reflexionsprofils in der Pupille zwischen einem LED-Feld "innerhalb" des Retinoskops sowie einem zusätzlichen LED-Feld "außerhalb" des Retinoskops verglichen wird. Dieser Vergleich dient zur Feststellung (anhand der Profile), ob sich der Proband innerhalb des Messbereichs des Retinoskops, z.B. im Bereich von +5 Dioptrien bis -7 Dioptrien, befindet. Für diese Art der Messbereichskontrolle kann z.B. ein Muster des Leuchtdiodenfeldes mit 5 Leuchtdioden der inneren beiden Zeilen gegenüber vier Leuchtdioden der äußeren beiden Zeilen bzw. im Vergleich zum ganzen Leuchtdiodenfeld zum Einsatz kommen.

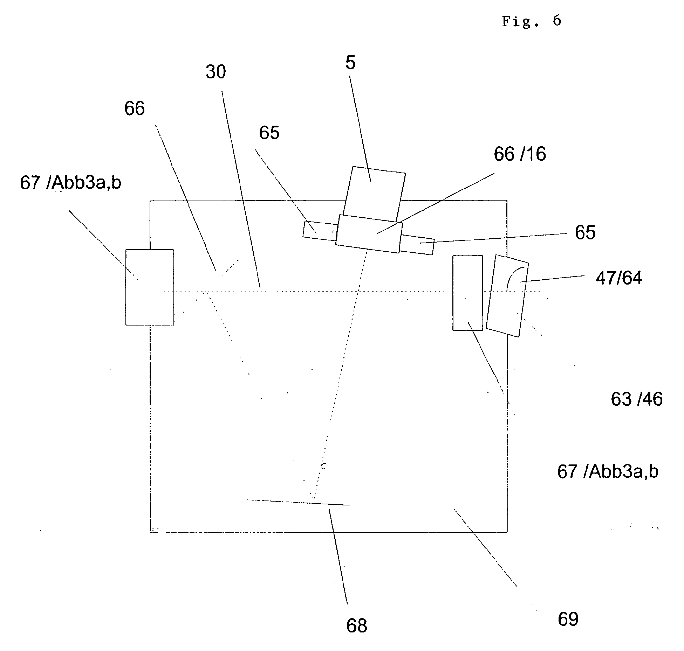

Fig. 6 zeigt die beschriebene Vorrichtung zur Refraktionsmessung innerhalb eines kompakten Gehäuses 69. Dabei wurde die Messstrecke beispielhaft durch zwei Spiegel 68/66 innerhalb des Gehäuses 69 realisiert. Denkbar ist auch die Realisierung der Messstrecke oder der Targetstrecke durch mehr oder weniger als zwei Spiegel. Die Spiegel können dabei über unterschiedliche reflektive Eigenschaften bezüglich der Wellenlänge des Lichtes verfügen. Zumindest reflektieren die Spiegel der Messstrecke IR-Licht. Ein Einblicksfenster 46/64 besteht aus einem Plangas und ist wahlweise nicht reflektiv für IR-Licht oder ist gegenüber der optischen Achse um einen Winkel 47/64 verkippt. Ebenso ist es denkbar, dass hier wahlweise ein refraktives Element anbringbar ist.

Das Gerät beinhaltet ein Targetsystem 67 gemäß Fig. 3a oder 3b zum "fogging" bzw. zur Provokation der Akkommodation, das sowohl eingespiegelt oder auch eingebracht werden kann. Im Falle der Einspiegelung sind die Spiegel für IR-Licht nicht reflektiv. Für den Fall der Präsentation des Objektes in der Position 67 ist der Spiegel 66 für IR reflektiv und für sichtbares Licht nicht reflektiv. Das Gehäuse 69 ist innenseitig beleuchtbar zur Beeinflussung der Pupillengröße. Ebenso ist es denkbar, die Helligkeit des Targets zur Beeinflussung der Pupillengröße zu steuern. Da die Kamera 5 bzw. der Messkopf die Pupillengröße erfasst und das System in Echtzeit die Pupillengröße berechnet, ist es möglich, die Pupillengröße dynamisch während des Messvorganges durch Änderung der Beleuchtungsintensität zu steuern.

Weiterhin ist die Vorrichtung dadurch gekennzeichnet, dass der Messkopf mit Kamera 5 und Retinoskop 16 aus dem Gehäuse mechanisch entnehmbar ist. Damit kann mit dem Messkopf allein ebenso im freien Raum gemessen werden. Eine weitere Variante besteht darin, dass sich im Gehäuse 69 eine zusätzliche Beleuchtung 65 des Retinoskops befindet. Durch die Einbringung des Messkopfes in das Gehäuse 69 wird damit das Beleuchtungsfeld des Retinoskops radial ausgedehnt, was zu einer Messbereichserweiterung führt. Eine entsprechende Sensorik für die Aufnahme des Messkopfs meldet dies an das System.

Ebenso ist es möglich, die Messstrecke mit Hilfe einer "Fernrohroptik" im Inneren eines Gehäuses abzubilden. Mit Hilfe von Pluslinsen kann der Öffnungswinkel des Objektivs der Kamera kondensiert werden sowie mit einem Zerstreuungsglas wieder geöffnet werden. Es sind auch Kombinationen aus Spiegel und (Fernrohr-)Optik denkbar. Ebenso ist es möglich, die Beleuchtung des Messkopfes abzuschalten und eine entsprechende Beleuchtung mit Blende im Bereich des Einblickfensters 47 anzubringen oder einzuspiegeln.

## Patentansprüche

1. Vorrichtung zur Refraktionsbestimmung aus einer relativen Distanz mit einer Kamera (5, 51), einer Beleuchtungseinheit (25, 52), einer Blendeneinheit (26) und einer Steuereinheit (53, 54, 55), **dadurch gekennzeichnet, dass** die Steuereinheit (53, 54, 55) Mittel zur dynamischen Helligkeits- und/oder Beleuchtungszeiteinstellung für die Beleuchtungseinheit (25, 52) umfasst.

2. Vorrichtung zur Refraktionsbestimmung aus einer relativen Distanz, insbesondere nach Anspruch 1, mit einer Kamera (5, 51), einer Beleuchtungseinheit (25), einer Blendeneinheit (26) und einer Steuereinheit (53, 54, 55),
**dadurch gekennzeichnet dass**,
eine Distanzmesseinheit (7, 11) zur Erfassung der Patientendistanz vorgesehen ist.

3. Vorrichtung zur Refraktionsbestimmung aus einer relativen Distanz, insbesondere nach Anspruch 1 oder 2, mit einer Kamera (51), einer Beleuchtungseinheit 25), einer Blendeneinheit (26) und einer Steuereinheit (53, 54, 55),
**dadurch gekennzeichnet, dass**
Messbereichskontrollmittel vorgesehen sind, die feststellen, ob die Sehcharakteristik eines Probanden innerhalb eines vorgebbaren Refraktions-Messbereichs der Vorrichtung liegt.

4. Vorrichtung nach einem der Ansprüche 1 bis 3, weiter **dadurch gekennzeichnet, dass** die Beleuchtungseinheit (25, 52) zur Erzeugung eines Beleuchtungsfeldes mit bereichsweisen Intensitätsunterschieden ansteuerbar ist.

5. Vorrichtung nach einem der Ansprüche 1 bis 4, weiter **dadurch gekennzeichnet, dass** die Beleuchtungseinheit eine Anordnung von mehreren Einzellichtquellen beinhaltet, die zur Erzeugung eines oder mehrerer vorgebbarer Beleuchtungshelligkeitsmuster individuell ansteuerbar sind.

6. Vorrichtung nach einem der Ansprüche 2 bis 5, weiter **gekennzeichnet durch** Mittel zur Gewinnung von Refraktionsmessdaten in Abhängigkeit von Distanzdaten der Distanzmesseinheit (7, 11).

7. Vorrichtung nach einem der Ansprüche 1 bis 6, weiter **gekennzeichnet durch** eine Akkommodationsbeeinflussungsoptik.

8. Verfahren zur Refraktionsbestimmung aus einer relativen Distanz unter Verwendung einer Kamera (5, 51), einer Beleuchtungseinheit (25), einer Blendeneinheit (26) und einer Steuereinheit (53, 54, 55),
**dadurch gekennzeichnet, dass**
die Helligkeit und/oder die Beleuchtungszeit der Beleuchtungseinheit (25, 52) dynamisch eingestellt wird.

9. Verfahren zur Refraktionsbestimmung aus einer relativen Distanz unter Verwendung einer Kamera (5, 51), einer Beleuchtungseinheit (25), einer Blendeneinheit (26) und einer Steuereinheit (53, 54, 55),
**dadurch gekennzeichnet, dass**
die Patientendistanz durch eine Distanzmesseinheit (7, 11) erfasst wird.

10. Verfahren zur Refraktionsbestimmung aus einer relativen Distanz unter Verwendung einer Kamera (5, 51), einer Beleuchtungseinheit (25), einer Blendeneinheit (26) und einer Steuereinheit (53, 54, 55),
**dadurch gekennzeichnet, dass**
eine selbsttätige Messbereichskontrolle durchgeführt wird, bei der festgestellt wird, ob die Sehcharakteristik eines Probanden innerhalb eines vorgebbaren Refraktions-Messbereichs liegt.
